# EUROPEAN PATENT APPLICATION

(11) **EP 4 427 662 A1**
(43) Date of publication of application: **11.09.2024**
(21) Application number: 24161734.9
(22) Date of filing: 06.03.2024
(51) Int. Cl.: A61B 1/018, A61B 17/34, A61B 8/08, A61B 1/00

(54) **PUNCTURE DEVICE**

(30) Priority: 06.03.2023 JP 2023033591
(71) Applicant: FUJIFILM Corporation, Tokyo 106-8620 (JP)
(72) Inventor: ONO, Hirotoshi, Kanagawa, 258-8538 (JP)
(74) Representative: Kudlek, Franz Thomas

(57) **Abstract**

A puncture device includes: a puncture needle (7); and an operation part that moves the puncture needle in an axial direction and is attachable to and detachable from an ultrasonic endoscope (93). The operation part includes a gripping member (10) that is interlocked with the puncture needle and whose outer peripheral surface is exposed, a first member (11) that is connected to the gripping member and is movable relative to the gripping member in the axial direction, and a restricting member (20) that restricts a relative movable maximum distance between the first member and the gripping member, and the outer peripheral surface of the gripping member has a shape including a pair of first planes that are arranged in a first direction and are disposed to face each other, and a pair of second planes that are arranged in a second direction intersecting the first direction and are disposed to face each other.

## Description

### BACKGROUND OF THE INVENTION

### 1. Field of the Invention

The present invention relates to a puncture device.

### 2. Description of the Related Art

JP2007-236684A describes a puncture device in which a puncture member consisting of a puncture needle and a flexible tube connected to the puncture needle is configured to move along a slide shaft and a foremost position of the slide shaft is restricted by a restricting ring. The slide shaft has a cylindrical shape on a rear end part side and a distal end side, which are exposed from a guide tube, and a non-exposed portion inside the guide tube is a square column.

WO2022/158405A describes a puncture needle comprising an outer needle having a lumen penetrating in an axial direction, an outer needle hub joined to a proximal end part of the outer needle, an inner needle inserted into the lumen of the outer needle via the outer needle hub, a switching mechanism configured to switch between a first state in which a needle tip is sharp and a second state in which the needle tip is blunt by displacing the inner needle in the axial direction, and a locking mechanism that maintains a position of the inner needle.

JP2015-142620A describes an organ fixation device comprising a fixture that has a locking portion and a suture connected to the locking portion and that fixes an organ, an accommodation portion that accommodates the locking portion, a housing that supports the accommodation portion, and a holding band that is made of a non-rubber resin material and that surrounds an outer periphery of the housing, in which the suture is led out from the accommodation portion, positioned along a periphery of the housing, sliding contact with the holding band and the housing, and held to be withdrawable.

JP2019-097907A describes an endoscopic snare including a flexible sheath, two parallel cables that slide within the sheath, an elastic wire of which both ends are connected to respective distal ends of the two cables and that exits and retracts from the sheath to form a loop in a case of protruding, and a proximal operation part for operating an exit/retraction of the wire and expansion/contraction of the loop via the cables, in which the wire includes two bending portions that are habitually formed on a proximal end side and a distal end side at a predetermined interval and that are provided at positions near a sheath distal end in a case where the wire forms the loop, the positions being positioned at a proximal end part (distal end) on one side of the loop, and the proximal operation part includes first sliding means for sliding both the parallel cables and second sliding means for sliding only the cable to which an wire end part on a side where the bending portion is not provided is connected.

JP2006-506200A describes a medical instrument including a housing, a probing needle that has a portion inside the housing, a cannula that concentrically accepts the probing needle and has a portion inside the housing, and a member combined with the housing.

JP2005-529632A describes a biopsy device that is used to extract a tissue sample and that consists of a handpiece into which a hollow biopsy needle is inserted, in which the biopsy needle includes a sample extraction chamber, a part of a portion of the biopsy needle, which protrudes from the handpiece, is inserted into a tissue to be examined, and the tissue is suctioned into the sample extraction chamber by vacuum, then separated by a sample separation device, and subsequently extracted.

### SUMMARY OF THE INVENTION

A puncture device that is used by being mounted on an ultrasonic endoscope enables a puncture needle to protrude from a distal end and reach a target site through a movement operation of a movable portion provided in an operation part. Therefore, it is important to facilitate the movement operation of the movable portion in order to realize an appropriate puncture procedure.

An object of the present disclosure is to provide a puncture device capable of realizing an appropriate puncture procedure.

According to an embodiment of the technology of the present disclosure, there is provided a puncture device comprising: a puncture needle; and an operation part that moves the puncture needle in an axial direction and is attachable to and detachable from an ultrasonic endoscope, in which the operation part includes a gripping member that is interlocked with the puncture needle, a first member that is connected to the gripping member and is movable relative to the gripping member in the axial direction, and a restricting member that restricts a relative movable distance between the first member and the gripping member, and an outer peripheral surface of the gripping member includes a pair of first planes that are arranged in a first direction and are disposed to face each other, and a pair of second planes that are arranged in a second direction intersecting the first direction and are disposed to face each other.

According to another embodiment of the technology of the present disclosure, there is provided a puncture device comprising: a puncture needle; and an operation part that moves the puncture needle in an axial direction and is attachable to and detachable from an ultrasonic endoscope, in which the operation part includes a gripping member that is interlocked with the puncture needle, a first member that is connected to the gripping member and is movable relative to the gripping member in the axial direction, and a restricting member that restricts a relative movable distance between the first member and the gripping member, the restricting member includes a movable member that is attached to an outer peripheral surface of the first member to be movable along the axial direction with respect to the first member, and a first rotational movement member that is rotationally movable with respect to the movable member, and an outer peripheral surface of the first rotational movement member includes a pair of planes that are disposed to face each other, and a pair of planes that are arranged in a direction intersecting a direction in which the pair of facing planes are arranged and that are disposed to face each other.

According to the technology of the present disclosure, it is possible to realize an appropriate puncture procedure.

### BRIEF DESCRIPTION OF THE DRAWINGS

Fig. 1 is a schematic view showing an ultrasonic endoscope to which a puncture device is attached.
Fig. 2 is a perspective view of the puncture device.
Fig. 3 is a longitudinal cross-sectional view of the puncture device showing a distal end portion in an enlarged manner.
Fig. 4 is a schematic view showing an example of a method of using the ultrasonic endoscope.
Fig. 5 is a perspective view showing an operation example of an operation part of the puncture device.
Fig. 6 is a perspective view showing an operation example of the operation part of the puncture device and is a perspective view showing a state in which a gripping member is moved from a state shown in Fig. 5.
Fig. 7 is an explanatory view of a gripping form of the gripping member shown in Fig. 5 as viewed from an axial direction.
Fig. 8 is a perspective view showing another operation example of the operation part of the puncture device.
Fig. 9 is a perspective view showing a state in which the gripping member is moved from a state shown in Fig. 8.
Fig. 10 is an explanatory view of a gripping form of the gripping member shown in Fig. 8 as viewed from the axial direction.
Fig. 11 is a perspective view showing still another operation example of the operation part of the puncture device.
Fig. 12 is a perspective view showing a state in which the gripping member is moved from a state shown in Fig. 11.
Fig. 13 is an explanatory view of a gripping form of the gripping member shown in Fig. 11 as viewed from the axial direction.
Fig. 14 is a perspective view showing still another operation example of the operation part of the puncture device.
Fig. 15 is a perspective view showing a state in which the gripping member is moved from a state shown in Fig. 14.
Fig. 16 is an explanatory view of a gripping form of the gripping member shown in Fig. 14 as viewed from the axial direction.
Fig. 17 is a perspective view showing an attachment state of the puncture device to the ultrasonic endoscope.
Fig. 18 is an explanatory view showing a form of gripping and operating a first rotational movement member of a restricting member.

### DESCRIPTION OF THE PREFERRED EMBODIMENTS

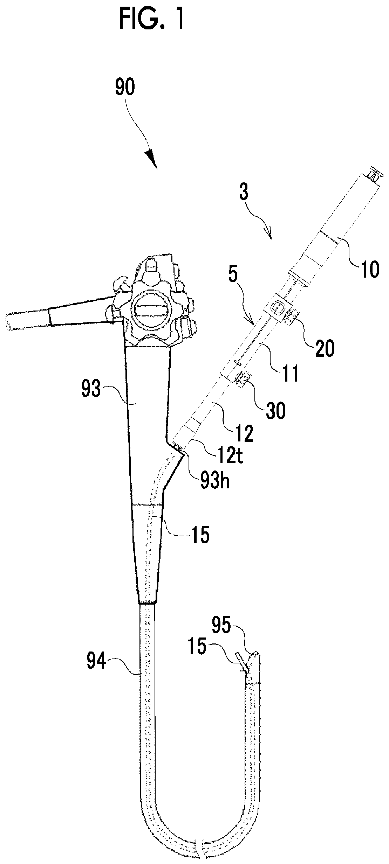

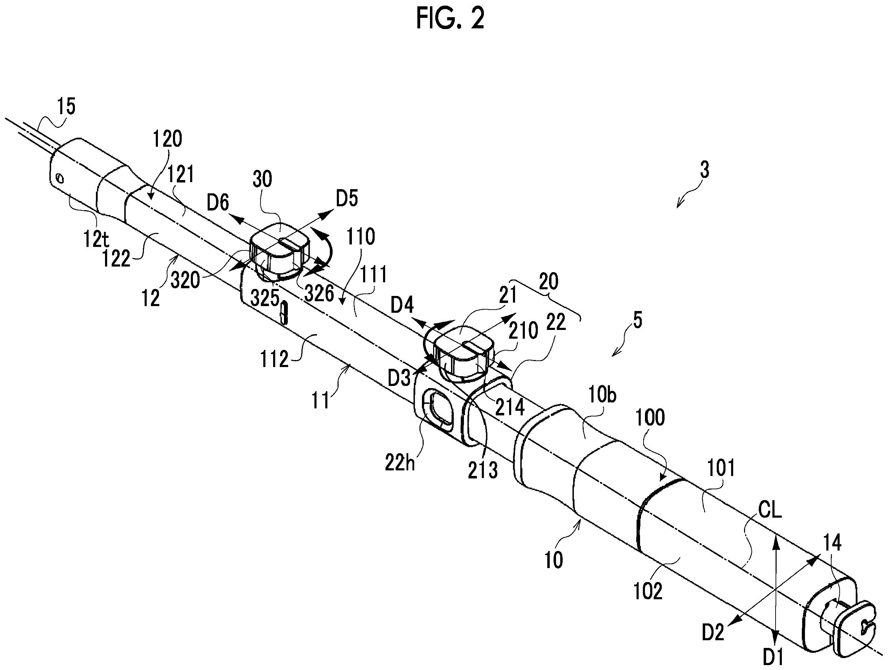

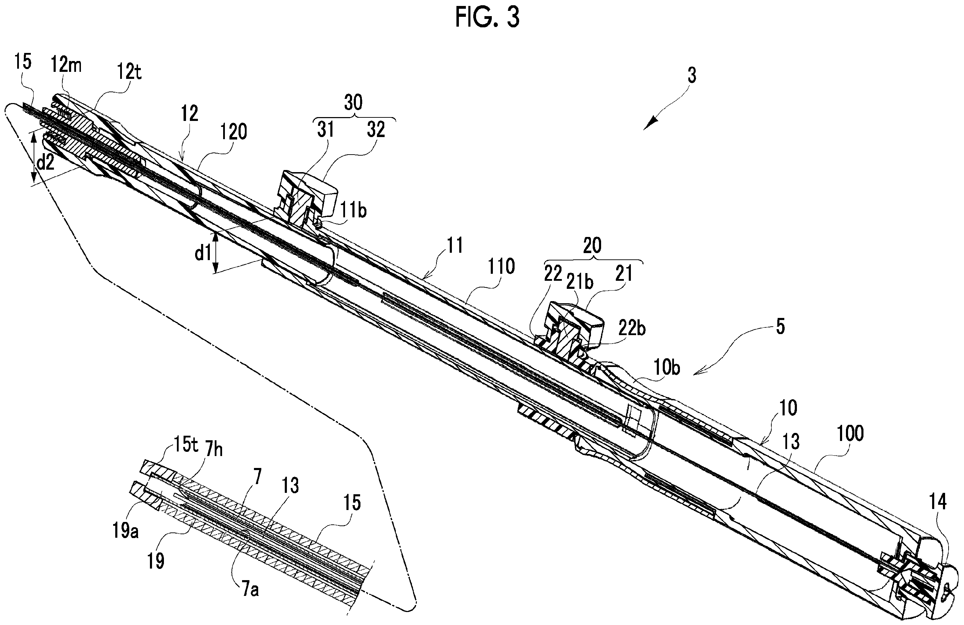

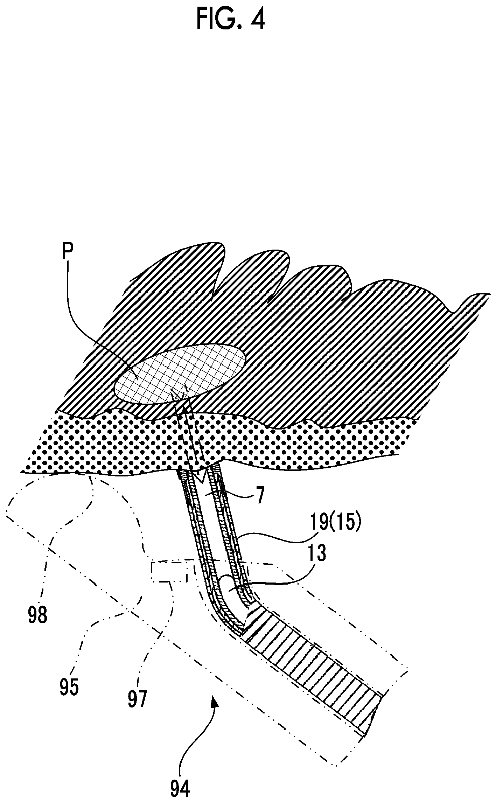

Fig. 1 is a schematic view showing an ultrasonic endoscope 90 to which a puncture device 3, which is an embodiment of the technology of the present disclosure, is attached. Fig. 2 is a perspective view of the puncture device 3. Fig. 3 is a longitudinal cross-sectional view of the puncture device 3 showing a distal end portion in an enlarged manner. Fig. 4 is a schematic view showing an example of a method of using the ultrasonic endoscope 90.

The ultrasonic endoscope 90 comprises an endoscope insertion part 94 that is inserted into a body and that includes an optical imaging mechanism 97 and an ultrasonic scanning mechanism 98 (see Fig. 4) provided in a distal end part 95, and an endoscope operation part 93 consecutively provided at a proximal end of the endoscope insertion part 94. The endoscope operation part 93 is provided with a treatment tool introduction hole 93h. The puncture device 3 can be mounted on the treatment tool introduction hole 93h.

The puncture device 3 is used by being mounted on the ultrasonic endoscope 90, for example, in order to collect a tissue inside a living body. The puncture device 3 comprises an elongated and tubular operation part 5, and an elongated insertion body 15 of which a proximal end side is built in the operation part 5. The insertion body 15 is inserted into a treatment tool channel of the endoscope insertion part 94 via the treatment tool introduction hole 93h. By operating the operation part 5, a distal end of the insertion body 15 can be advanced and retracted from the distal end part 95 of the ultrasonic endoscope 90 as shown in Fig. 4. Hereinafter, a direction in which an axis CL (see Fig. 2) of the operation part 5 extends is referred to as an axial direction. Among both ends of the puncture device 3 in the axial direction, a side close to the ultrasonic endoscope 90 in a state of being mounted on the ultrasonic endoscope 90 is referred to as a distal end, and a side far from the ultrasonic endoscope 90 is referred to as a proximal end.

As shown in Fig. 3, the operation part 5 of the puncture device 3 has a tubular configuration having an internal space. The operation part 5 comprises a tubular gripping member 10, a tubular first member 11 that is inserted into an inside of the gripping member 10 from a distal end side of the gripping member 10 and that is configured to move relative to the gripping member 10 in the axial direction, a tubular second member 12 that is inserted into an inside of the first member 11 from a distal end side of the first member 11 and that is configured to move relative to the first member 11 in the axial direction. A stylet 13 is attached to a proximal end part of the gripping member 10 via a proximal end fixing member 14.

A distal end part 12t of the second member 12 is mounted on and fixed to the treatment tool introduction hole 93h. The puncture device 3 is configured such that the first member 11 and the gripping member 10 are slidably movable in the axial direction with respect to the second member 12 fixed to the endoscope operation part 93.

As shown in the enlarged portion of Fig. 3, the insertion body 15 is configured with an outer layer side made of a flexible sheath 19 consisting of, for example, an adherent coil or the like. A tube-like puncture needle 7 is provided in an insertion path 19a inside the flexible sheath 19. The puncture needle 7 has a sharp distal end shape, and can be switched between a state in which the distal end is retracted inside the flexible sheath 19 and a state in which the distal end protrudes from a distal end part 15t of the flexible sheath 19, through the operation of the operation part 5. An opening portion 7h serving as an entrance for a punctured tissue to enter the inside is formed at a distal end of the puncture needle 7. The stylet 13 supported by the proximal end fixing member 14 fixed to the proximal end part of the operation part 5 is inserted into an inside 7a of the puncture needle 7.

The material of the puncture needle 7 is preferably manufactured from a member that has flexibility and elasticity that allows the puncture needle 7 to easily return to a linear state even in a case of being bent by an external force. For example, the puncture needle 7 is manufactured by using an alloy material such as a stainless steel alloy, a nickel-titanium alloy, or a cobalt-chromium alloy.

As shown in Fig. 2, the gripping member 10 has a shape in which an outer peripheral surface 100 thereof has a plane. Fig. 2 shows a first direction D1 and a second direction D2 orthogonal to the first direction D1, as two directions perpendicular to the axis CL.

The outer peripheral surface 100 of the gripping member 10 has a shape including a pair of first planes 101 (not shown on a lower surface side) that are arranged in the first direction D1 and are disposed to face each other, and a pair of second planes 102 (not shown on a background side of the paper) that are arranged in the second direction D2 and are disposed to face each other. The first plane 101 is a plane perpendicular to the first direction D1, and the second plane 102 is a plane perpendicular to the second direction D2.

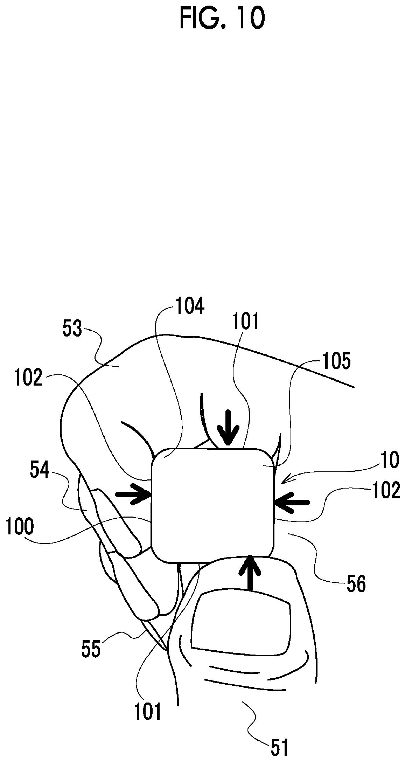

In the example of Fig. 2, the first direction D 1 and the second direction D2 are orthogonal to each other, but the first direction D1 and the second direction D2 need only intersect each other. As shown in Fig. 10, which will be described below, it is preferable that lengths of four planes, that is, the pair of first planes 101 and the pair of second planes 102, are substantially the same in a state in which the gripping member 10 is viewed in the axial direction. Two lengths being substantially the same means that the two lengths coincide with each other, excluding tolerances in difference.

As shown in Fig. 10, a cross-sectional outer shape of the gripping member 10 perpendicular to the axial direction (hereinafter, simply referred to as a cross-sectional shape of the gripping member 10) is preferably a shape in which four corner portions of a square are chamfered in a curved shape. The cross-sectional shape of the gripping member 10 may be a square or a shape in which four corner portions of the square are chamfered in a linear shape. In addition, the cross-sectional shape of the gripping member 10 may be a rectangular shape or a shape in which four corner portions of the rectangular shape are chamfered. As described above, the cross-sectional shape of the gripping member 10 is a quadrangle or a shape in which four corner portions of the quadrangle are chamfered.

As shown in Fig. 2, a sunken portion 10b that is sunken inward from other portions of the gripping member 10 is provided on the distal end side of the outer peripheral surface 100 of the gripping member 10. At any position in the axial direction, a cross-sectional shape of the sunken portion 10b, which is perpendicular to the axial direction, is a quadrangle or a shape in which four corner portions of the quadrangle are chamfered, similar to the outer peripheral surface 100.

The first member 11 is provided to be relatively movable in the axial direction within the gripping member 10. As shown in Fig. 2, an outer peripheral surface 110 of the first member 11 has preferably a shape including a pair of first planes 111 (not shown on the lower surface side) that are arranged in the first direction D1 and are disposed to face each other, and a pair of second planes 112 (not shown on the background side of the paper) that are arranged in the second direction D2 and are disposed to face each other. As shown in Fig. 2, the first plane 111 is preferably parallel to the first plane 101, and the second plane 112 is preferably parallel to the second plane 102.

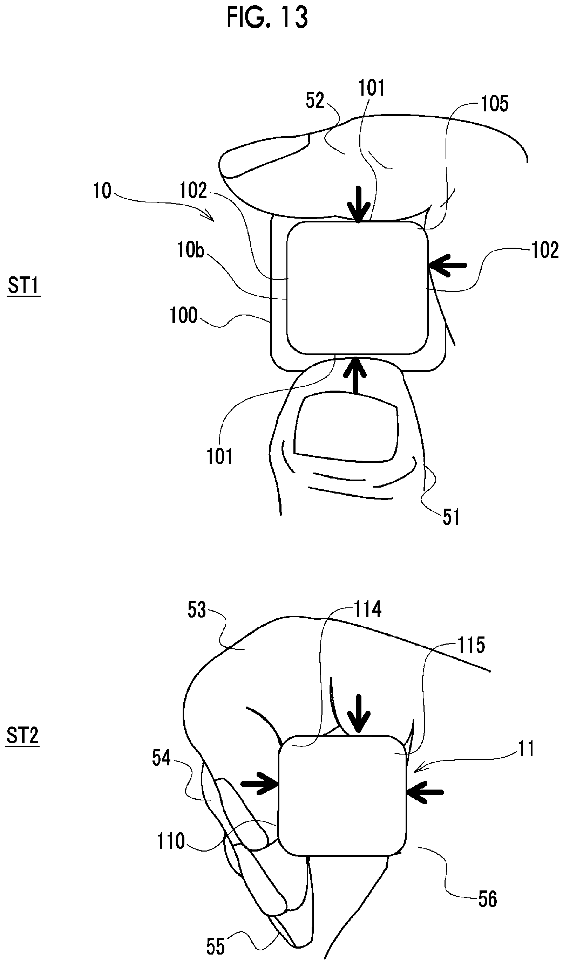

As shown in a state ST2 of Fig. 13, which will be described below, it is preferable that lengths of four planes, that is, the pair of first planes 111 and the pair of second planes 112 are substantially the same in a state in which the first member 11 is viewed in the axial direction. As described above, a cross-sectional outer shape of the first member 11 perpendicular to the axial direction is preferably a quadrangle or a shape in which corner portions of the quadrangle are chamfered, or is more preferably a square or a shape in which corner portions of the square are chamfered, similar to the gripping member 10.

A proximal end side of the flexible sheath 19 is fixed inside the first member 11. A proximal end side of the puncture needle 7 protrudes from the proximal end side of the flexible sheath 19 and is fixed inside the gripping member 10. Therefore, by sliding the gripping member 10 in the axial direction with respect to the first member 11 (by moving the gripping member 10 and the first member 11 relative to each other in the axial direction), it is possible to move the puncture needle 7 with respect to the flexible sheath 19 and cause the puncture needle 7 to project from the distal end of the flexible sheath 19.

The outer peripheral surface 110 of the first member 11 is provided with a restricting member 20 that restricts a relative movable maximum distance between the first member 11 and the gripping member 10 in the axial direction.

As shown in Figs. 2 and 3, the restricting member 20 comprises a movable member 22 that is attached to be slidably movable in the longitudinal direction of the first member 11 on the outer peripheral surface 110 of the first member 11, and a first rotational movement member 21 that is rotationally movable with respect to the movable member 22. That is, the movable member 22 is attached to the outer peripheral surface 110 to be slidably movable in the axial direction with respect to the first member 11.

The first rotational movement member 21 comprises, for example, a screw portion 21b extending in a direction (in the first direction D1 in the examples shown in Figs. 2 and 3) intersecting the axis CL. The screw portion 21b is screwed into and penetrates through an attachment portion 22b of the movable member 22. Therefore, a distal end portion of the screw portion 21b is configured to come into contact with the outer peripheral surface 110. In addition, the movable member 22 is provided with graduation opening portion 22h (see Fig. 2). The graduation opening portion 22h is, for example, for visually recognizing graduations indicating distances marked on one surface of the outer peripheral surface 110 of the first member 11 along the axial direction.

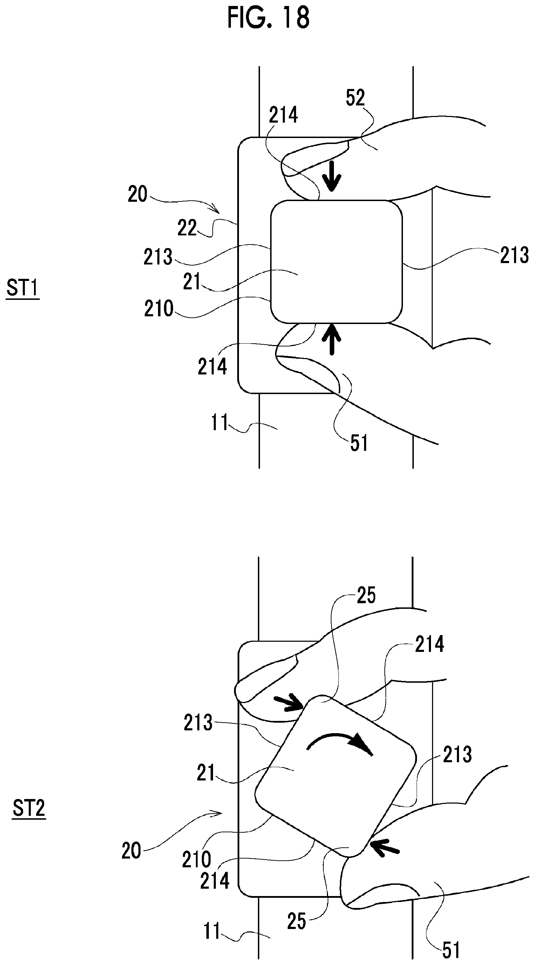

The first rotational movement member 21 is configured such that an upper end side of the screw portion 21b is a large-diameter portion. As shown in Fig. 18, an outer peripheral surface 210 (a side surface around a rotational movement shaft) of the large-diameter portion of the first rotational movement member 21 has a shape including a pair of third planes 213 that are arranged in a third direction D3 indicating the same direction as the second direction D2 in Fig. 2 and are disposed to face each other, and a pair of fourth planes 214 that are arranged in a fourth direction D4 intersecting (in the present embodiment, orthogonal to) the third direction D3 and are disposed to face each other. That is, the outer peripheral surface 210 of the first rotational movement member 21 has preferably a quadrangle or a shape in which corner portions of the quadrangle are chamfered in a plan view along a direction of the rotational movement shaft (a direction perpendicular to the third direction D3 and the fourth direction D4).

The restricting member 20 is configured to position a rotational movement position of the first rotational movement member 21 at a locked position to press the distal end portion of the screw portion 21b against the outer peripheral surface 110, thereby locking the movement of the movable member 22 with respect to the first member 11. On the other hand, the restricting member 20 is configured to rotate the first rotational movement member 21 from the locked position to separate the distal end portion of the screw portion 21b from the outer peripheral surface 110, thereby unlocking the movement of the movable member 22 with respect to the first member 11. Fig. 3 shows a state in which the restricting member 20 is positioned on a most proximal end side within a movement range in the axial direction. In a case where the restricting member 20 is moved to the distal end side from the state of Fig. 3 and is locked in that state, the distance in the axial direction between the restricting member 20 and the gripping member 10 can be fixed to a first distance. In this state, by slidably moving the gripping member 10 toward the first member 11, the distal end of the puncture needle 7 fixed to the gripping member 10 can be moved by the first distance at most.

The second member 12 is provided to be relatively movable in the axial direction within the first member 11. As shown in Fig. 2, an outer peripheral surface 120 of the second member 12 has preferably a shape including a pair of first planes 121 (not shown on the lower surface side) that are arranged in the first direction D1 and are disposed to face each other, and a pair of second planes 122 (not shown on the background side of the paper) that are arranged in the second direction D2 and are disposed to face each other. As shown in Fig. 2, the first plane 121 is preferably parallel to the first plane 101, and the second plane 122 is preferably parallel to the second plane 102.

It is preferable that lengths of four planes, that is, the pair of first planes 121 and the pair of second planes 122, are substantially the same in a state in which the second member 12 is viewed in the axial direction. In other words, a cross-sectional outer shape of the second member 12 perpendicular to the axial direction is preferably a quadrangle or a shape in which corner portions of the quadrangle are chamfered, or is more preferably a square or a shape in which corner portions of the square are chamfered, similar to the gripping member 10.

The distal end part 12t of the second member 12 is configured to have a diameter larger than that of other portions that can be inserted into the inside of the first member 11. In other words, an outer diameter d2 (see Fig. 3) of the distal end part 12t is configured to be larger than an inner diameter d1 of the first member 11. The distal end part 12t is provided with a luer lock 12m (see Fig. 3) that can be mounted on a socket of the treatment tool introduction hole 93h of the ultrasonic endoscope 90.

The second member 12 is attached to the endoscope operation part 93 of the ultrasonic endoscope 90. Therefore, the position of the second member 12 in the axial direction remains unchanged in a state in which the puncture device 3 is mounted on the ultrasonic endoscope 90. On the other hand, in this state, the first member 11 is slidable with respect to the second member 12 in the axial direction. By changing a relative position in the axial direction between a distal end edge of the first member 11 and a distal end edge of the second member 12 (hereinafter, simply referred to as a relative position between the first member 11 and the second member 12), a protruding amount of the flexible sheath 19, which is fixed to the first member 11, from the distal end part 95 of the endoscope insertion part 94 can be changed.

The relative position between the first member 11 and the second member 12 can be fixed to any of a plurality of positions by using a fixing member 30 provided at the distal end part of the first member 11.

The fixing member 30 comprises a second rotational movement member 32 that is rotationally movable integrally with a screw portion 31 (see Fig. 3), which is screwed into and penetrates through a distal end attachment portion 11b of the first member 11, about the screw portion 31. The distal end portion of the screw portion 31 is configured to come into contact with the outer peripheral surface 120 of the second member 12.

The second rotational movement member 32 is configured such that an upper end side of the screw portion 31 is a large-diameter portion. An outer peripheral surface 320 (a side surface around a rotational movement shaft) of the large-diameter portion of the second rotational movement member 32 has a shape including a pair of fifth planes 325 that are arranged in a fifth direction D5 indicating the same direction as the second direction D2 in Fig. 2 and are disposed to face each other, and a pair of sixth planes 326 that are arranged in a sixth direction D6 intersecting (in the present embodiment, orthogonal to) the fifth direction D5 and are disposed to face each other. The outer peripheral surface 320 of the second rotational movement member 32 has preferably a quadrangle or a shape in which corner portions of the quadrangle are chamfered in a plan view along a direction of the rotational movement shaft (a direction perpendicular to the fifth direction D5 and the sixth direction D6).

The fixing member 30 is configured to position a rotational movement position of the second rotational movement member 32 at a locked position to press a distal end portion of the screw portion 31 against the outer peripheral surface 120, thereby fixing the position of the first member 11 with respect to the second member 12. On the other hand, the fixing member 30 is configured to rotate the second rotational movement member 32 from the locked position by, for example, 90 degrees and position the rotational movement position of the second rotational movement member 32 at an unlocked position to separate the distal end portion of the screw portion 31 from the outer peripheral surface 120, thereby releasing the first member 11 from the position fixation with respect to the second member 12.

For example, after the second rotational movement member 32 is rotated from the state shown in Fig. 2 and released from the fixation, and the distal end edge of the first member 11 is brought close to the distal end edge of the second member 12, the second rotational movement member 32 is rotated and fixed. In this state, the first member 11 fixed to the proximal end of the flexible sheath 19 moves to the distal end side. Therefore, it is possible to increase the protruding amount of the flexible sheath 19 from the distal end part 95 of the endoscope insertion part 94 as compared with the state shown in Fig. 2. As described above, by adjusting the relative position between the first member 11 and the second member 12 using the fixing member 30, it is possible to adjust the protruding amount of the flexible sheath 19 from the distal end part 95 of the endoscope insertion part 94.

Hereinafter, a method of using the puncture device 3 will be described with a biopsy treatment in which cells and the like of a lesion are collected by puncturing a lesion site inside a living body with the puncture needle 7, as an example.

First, an operator inserts the endoscope insertion part 94 into the body and introduces the distal end part 95 of the endoscope insertion part 94 to the vicinity of a target tissue P as shown in Fig. 4 while performing observation with the optical imaging mechanism 97. After the introduction, the operator determines a site to be subjected to the biopsy based on observation results by the optical imaging mechanism 97 and the ultrasonic scanning mechanism 98.

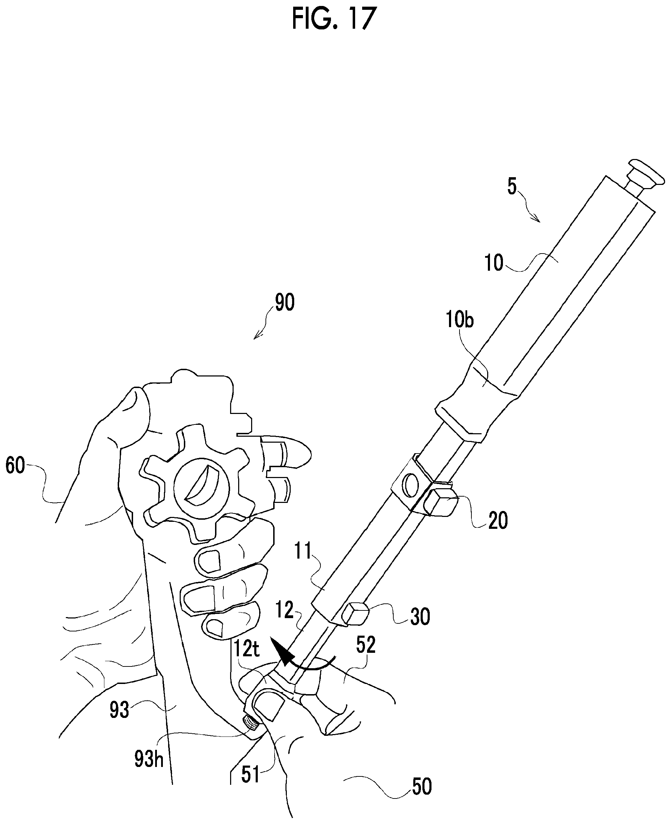

After determining the site, the operator takes the puncture device 3 in the hand and inserts the insertion body 15 located outside the distal end of the second member 12 into the treatment tool introduction hole 93h. Then, as shown in Fig. 17, the operator mounts the luer lock 12m of the distal end part 12t in the socket of the treatment tool introduction hole 93h while grasping the distal end part 12t of the second member 12 with their fingers. In this case, the operator rotates the operation part 5 while pinching the distal end part 12t with a thumb 51 and an index finger 52. The outer peripheral surface 120 of the distal end part 12t includes a pair of facing planes so that it is easy to apply a force to the distal end part 12t, which leads to good attachment operability.

Next, the operator rotates and unlocks the second rotational movement member 32 of the fixing member 30 and changes the relative position between the second member 12 and the first member 11 while observing the inside of the living body by using the optical imaging mechanism 97 and the ultrasonic scanning mechanism 98, to adjust the protruding amount of the flexible sheath 19 from the distal end part 95 to an appropriate amount. After this adjustment, the operator rotates the second rotational movement member 32 of the fixing member 30 to fix the relative position and fixes the protruding amount of the flexible sheath 19.

Next, the operator rotates and unlocks the first rotational movement member 21 of the restricting member 20 while taking into consideration a distance to the target tissue P as a biopsy target based on the observation result by the ultrasonic scanning mechanism 98 and moves the movable member 22. After that, the operator rotates the first rotational movement member 21 to the original position to fix the restricting member 20 to the first member 11. As a result, a maximum protruding length of the puncture needle 7 from the flexible sheath 19 is determined.

Next, for example, the operator pulls back the stylet 13 to the proximal end side of the puncture needle 7 as shown in Fig. 4 in a state in which the distal end of the flexible sheath 19 is in contact with the organ wall. Next, the operator slidably moves the gripping member 10 until the gripping member 10 comes into contact with the movable member 22. As a result, as shown in Fig. 4, the puncture needle 7 protrudes from the distal end of the flexible sheath 19, penetrates through the wall of the organ, and is pushed forward to reach the target tissue P. In this manner, the puncture procedure is performed.

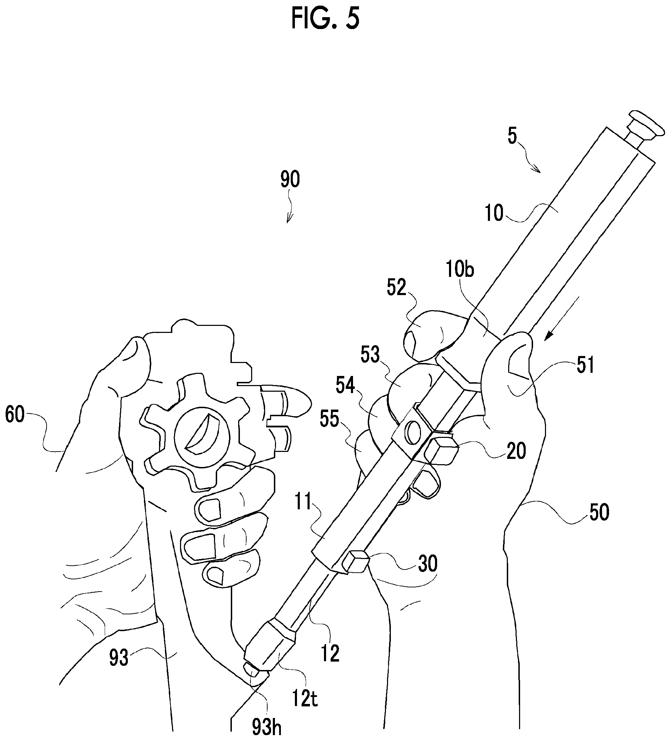

Hereinafter, an example of a method of holding the puncture device 3 will be described with reference to Figs. 5 to 16.

Figs. 5 to 7 are views illustrating a first operation method of the puncture device 3.

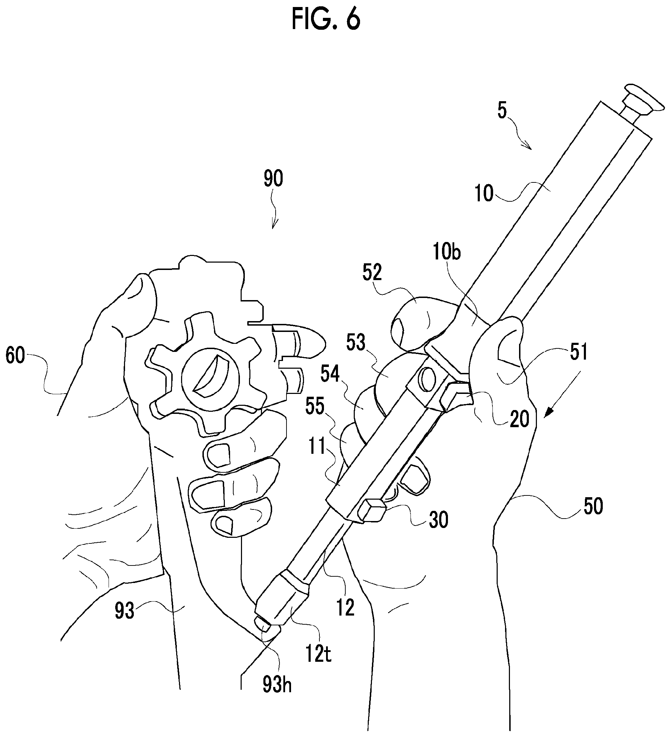

As shown in Fig. 5, the operator holds the endoscope operation part 93 with, for example, a left hand 60 and holds the operation part 5 of the puncture device 3 with a right hand 50. In the example shown in Fig. 5, the operator pinches the sunken portion 10b of the gripping member 10 with the thumb 51 and the index finger 52, and the other fingers (a middle finger 53, a ring finger 54, and a little finger 55) are not particularly specified but are kept away from or lightly placed on the first member 11, for example. Then, as shown in Fig. 6, the operator slides the gripping member 10 to approach the treatment tool introduction hole 93h and brings the distal end of the gripping member 10 into contact with the restricting member 20. As a result, the distal end of the puncture needle 7 protrudes from the flexible sheath 19 by a predetermined amount and punctures the target tissue P.

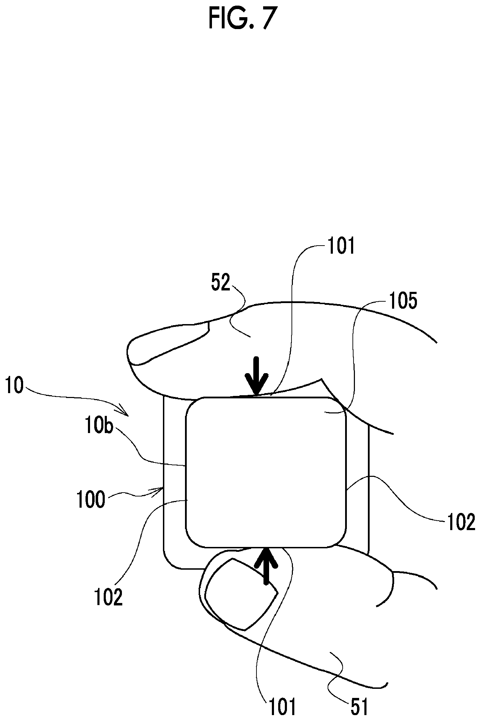

In this operation, the thumb 51 and the index finger 52 come into planar contact in a case of coming into contact with the first planes 101 of the outer peripheral surface 100 of the gripping member 10 as shown in Fig. 7. As described above, the puncture device 3 is configured such that a contact surface with the finger in the gripping member 10 is a plane, so that a force is easily transmitted to the gripping member 10 even in a case of gripping and moving the gripping member 10 with two fingers to move the puncture needle 7. As a result, it is possible to easily and appropriately conduct the puncture procedure.

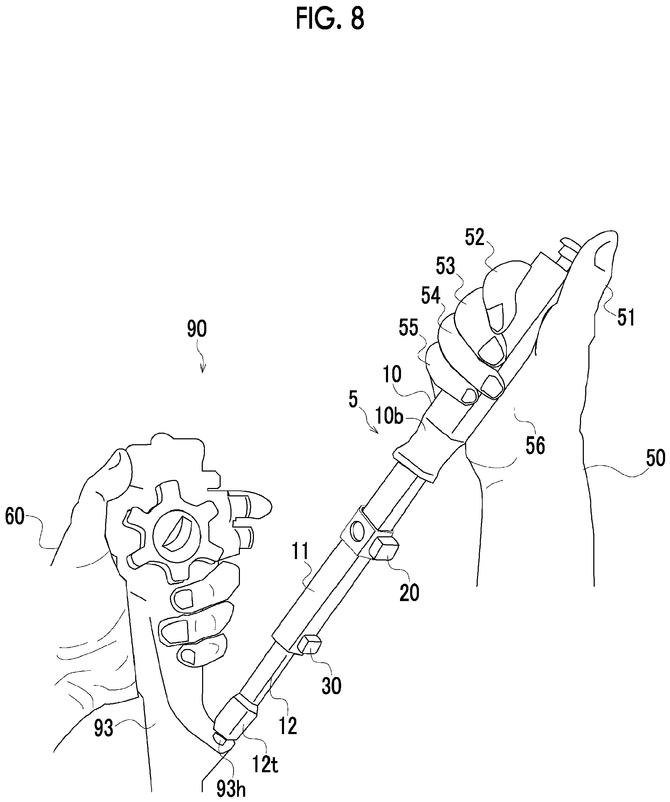

Figs. 8 to 10 are views illustrating a second operation method of the puncture device 3.

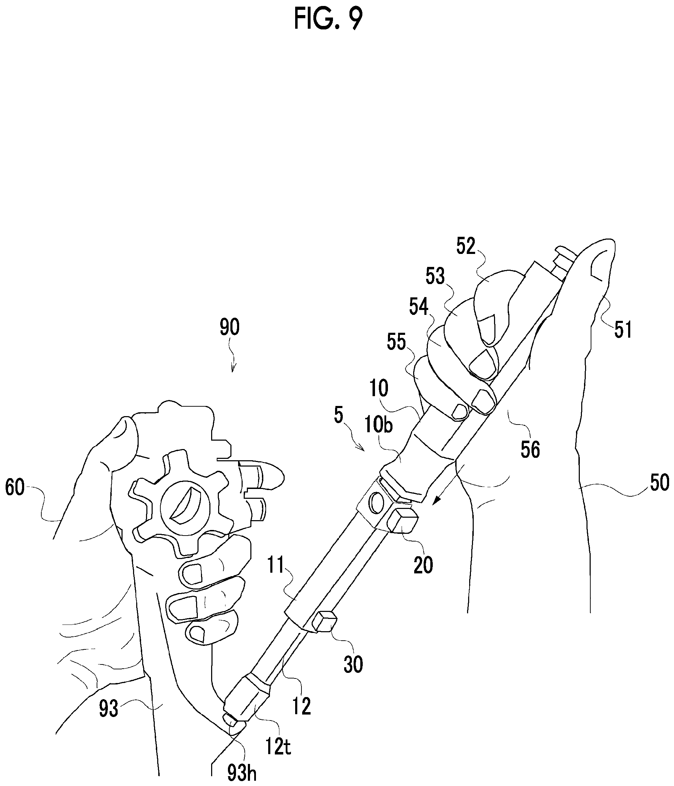

In the second operation method, the operator grips the gripping member 10 with the entire right hand 50 and slidably operates the gripping member 10, as shown in Figs. 8 and 9. For example, the operator places the thumb 51 along the axial direction against the gripping member 10 and uses a palm 56 to grip the gripping member 10 by winding the other four fingers around the outer peripheral surface 100 of the gripping member 10. In the second operation method, as shown in Fig. 10, the four planes of the outer peripheral surface 100 of the gripping member 10 can be pressed by the hand. Further, corner portions 104 and 105 of the gripping member 10 are in contact to be caught on the inner sides of the fingers. This makes it possible to more firmly grip the gripping member 10, which makes it easy to transmit a force to the gripping member 10. As a result, it is possible to easily and appropriately conduct the puncture procedure.

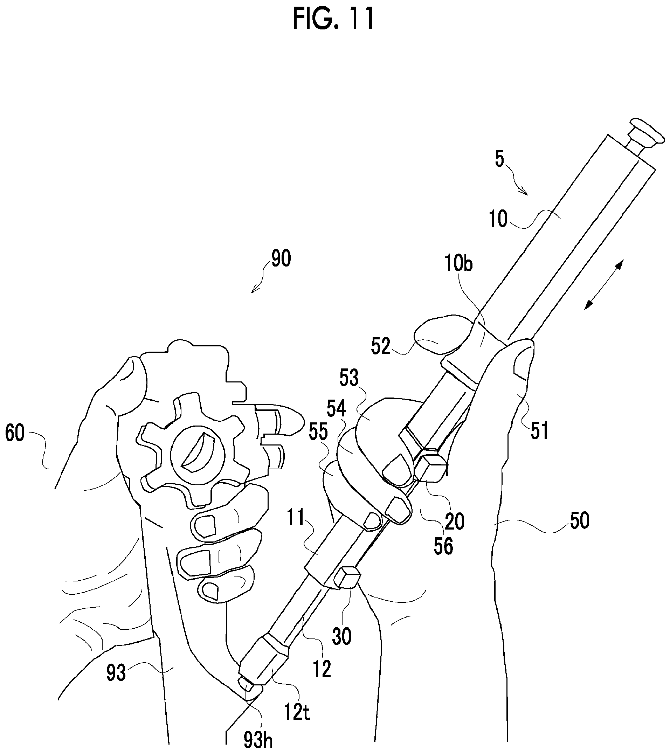

Figs. 11 to 13 are views illustrating a third operation method of the puncture device 3.

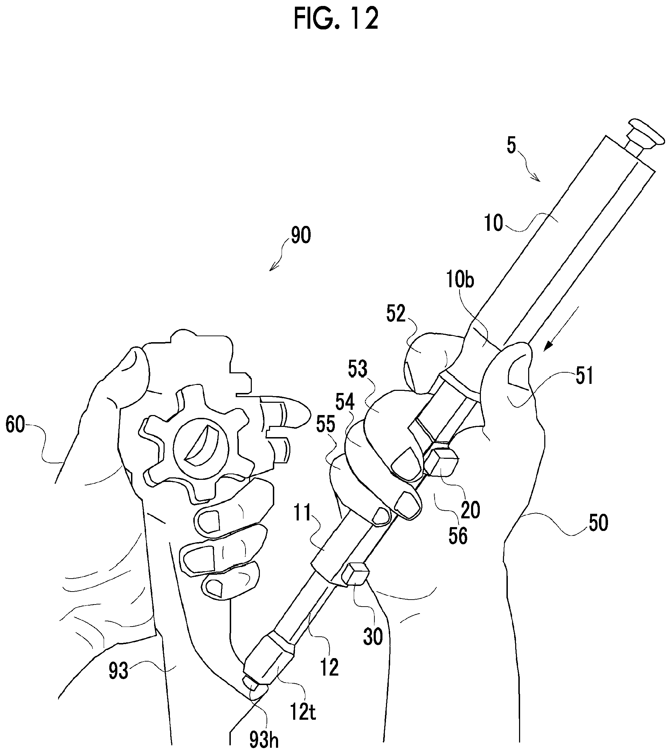

The third operation method is an operation assuming a case where, for example, a sample tissue is collected into a needle hole by reciprocating the puncture needle 7 that has punctured the target tissue P. In this case, the operator pinches the sunken portion 10b on the distal end side of the gripping member 10 with the thumb 51 and the index finger 52 and grips the first member 11 with the other fingers (the middle finger 53, the ring finger 54, and the little finger 55) and the palm 56. Then, the operator finely moves the gripping member 10 such that the thumb 51 and the index finger 52 are brought close to or away from the middle finger 53 as shown in Figs. 11 and 12.

In this case, since the thumb 51 and the index finger 52 are in planar contact with the first planes 101 of the outer peripheral surface 100 of the gripping member 10 as shown in a state ST1 of Fig. 13, the gripping member 10 can be gripped more firmly. In addition, the index finger 52 can be in contact to be caught on the corner portion 105 of the outer peripheral surface 100. Further, since the middle finger 53, the ring finger 54, and the little finger 55 are in contact such that a corner portion 114 and a corner portion 115 of the outer peripheral surface 110 of the first member 11 are caught on the inner sides of the fingers as shown in the state ST2 of Fig. 13, the first member 11 can be gripped more firmly. As a result, a force is easily transmitted to the gripping member 10, and it is possible to easily and appropriately conduct the puncture procedure.

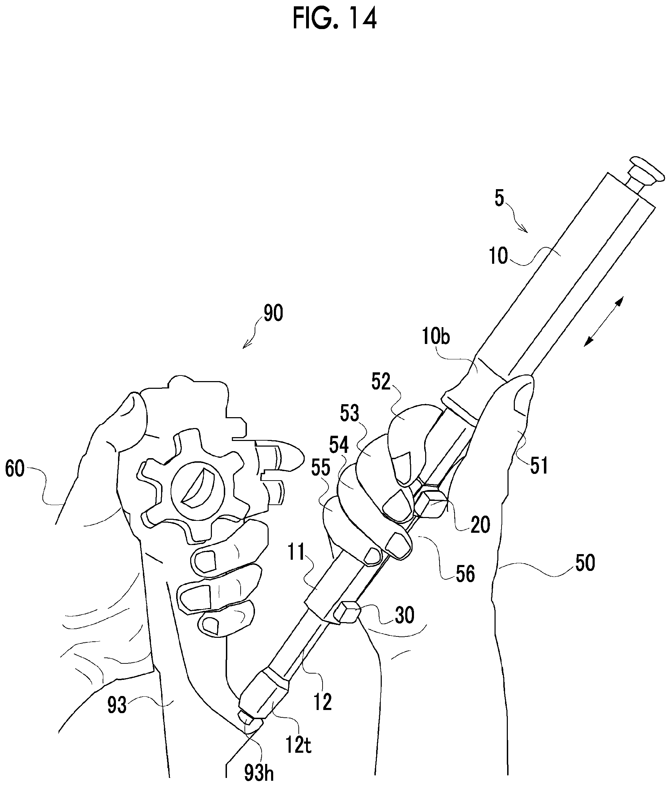

Figs. 14 to 16 are views illustrating a fourth operation method of the puncture device 3.

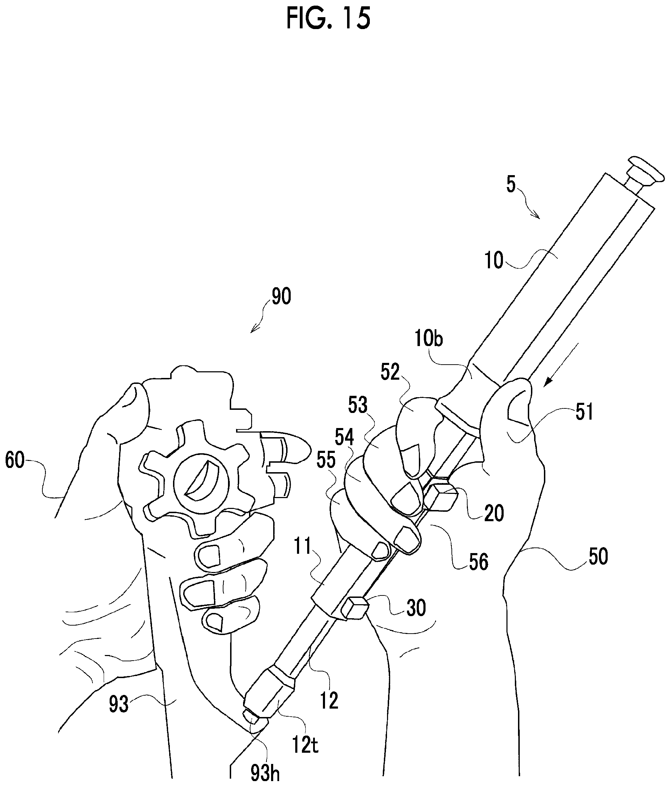

The fourth operation method is an operation assuming a case where the puncture needle 7 that has punctured the target tissue P is reciprocated. The operator places the thumb 51 on the sunken portion 10b of the gripping member 10 and grips the first member 11 with the other four fingers and the palm 56. Then, the operator moves the gripping member 10 by moving the thumb 51 in the axial direction as shown in Figs. 14 and 15.

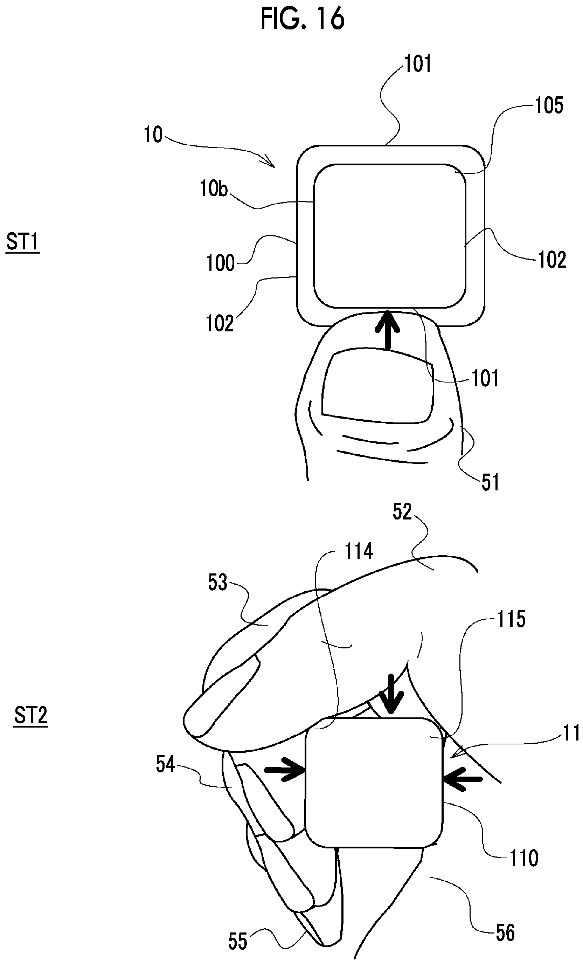

In this case, the thumb 51 is in contact with the first plane 101 of the outer peripheral surface 100 of the gripping member 10 as shown in a state ST1 of Fig. 16. Meanwhile, as shown in a state ST2 of Fig. 16, the other four fingers are in contact such that the corner portion 114 and the corner portion 115 of the outer peripheral surface 110 of the first member 11 are caught on the inner sides of the fingers. Further, since the pushing direction of the thumb 51 and the pushing direction of the other fingers are directions that face each other (facing directions), the first member 11 can be gripped more firmly. Therefore, it is possible to firmly press and move the gripping member 10 up and down by using the thumb 51. As a result, it is possible to easily and appropriately conduct the puncture procedure.

Fig. 18 is a view illustrating a method of operating the restricting member 20. Since the outer peripheral surface 210 of the first rotational movement member 21 has four planes as shown in Fig. 18, it is easy to apply a force because of planar contact as shown in a state ST1 when pinched with the thumb 51 and the index finger 52. Additionally, since corner portions 25 are caught on fingertips as shown in a state ST2 in a case where the first rotational movement member 21 is rotated, it is possible to firmly apply a force. The operation of the second rotational movement member 32 is the same as that of the first rotational movement member 21.

It is preferable that a direction in which the third plane 213 extends and the axial direction coincide with each other as shown in the state ST1 of Fig. 18 in a case where the rotational movement position of the first rotational movement member 21 is at the locked position. The first rotational movement member 21 may be in an unlocked state in a case where the rotational movement position is rotated slightly from the locked position, as shown in the state ST2 of Fig. 18. In such a case, with the puncture device 3, it is easy to distinguish between the locked state and the unlocked state because the first rotational movement member 21 has a quadrangle or a shape in which the corner portions of the quadrangle are chamfered in a plan view. For example, since the rotational movement position of the first rotational movement member 21 can be easily recognized as compared with a case where the plan view shape of the first rotational movement member 21 is circular, it is possible to prevent unintentional puncture by sliding the gripping member 10 in a forgotten locked state.

As described above, in the puncture device 3, the outer peripheral surface 110 of the first member 11 and the outer peripheral surface 100 of the gripping member 10 are parallel to each other. Therefore, for example, in a case of placing one hand on the parallel portion and gripping both the first member 11 and the gripping member 10, the grip can be easily performed. As a result, it is possible to improve the operability in a case where the first member 11 and the gripping member 10 are moved relative to each other.

Further, in the puncture device 3, the first member 11 and the gripping member 10 each have an outer peripheral surface shape having four planes. Therefore, for example, in a case of gripping both the first member 11 and the gripping member 10 with one hand, the grip can be easily performed. As a result, it is possible to improve the operability in a case where the first member 11 and the gripping member 10 are moved relative to each other.

Further, in the puncture device 3, the outer peripheral surface 120 of the second member 12 has a shape including four planes. Therefore, in a case where the distal end part 12t of the second member 12 is mounted on the treatment tool introduction hole 93h, the mounting can be stably performed.

In addition, in the puncture device 3, the outer diameter increases in the order of the second member 12, the first member 11, and the gripping member 10, which makes it easy to intuitively recognize a positional relationship between the first member 11 and the second member 12, and a positional relationship between the first member 11 and the gripping member 10, and the operability can be improved. Further, the gripping member 10 can be made sufficiently thick regardless of the size of the treatment tool introduction hole 93h of the ultrasonic endoscope 90, which makes it easy to move the puncture needle 7, and the operability can be improved. Moreover, since the outer peripheral surface 100 of the gripping member 10 having the largest outer diameter includes four planes, it is possible to prevent the operation part 5 from rolling in a case where the operation part 5 is placed on a placement surface such as a desk.

Additionally, in the puncture device 3, a length of each of the pair of first planes 101 and a length of each of the pair of second planes 102 coincide with each other in a state of being viewed in the axial direction. Therefore, a force can be easily transmitted to the gripping member 10 regardless of the grip posture of the gripping member 10.

As described so far, at least the following matters are described in the present specification. It should be noted that components and the like corresponding to the above-described embodiments are shown in parentheses, but the present invention is not limited thereto.
(1) A puncture device (puncture device 3) comprising:
   a puncture needle (puncture needle 7); and
   an operation part (operation part 5) that moves the puncture needle in an axial direction and is attachable to and detachable from an ultrasonic endoscope (ultrasonic endoscope 90),
   in which the operation part includes a gripping member (gripping member 10) that is interlocked with the puncture needle and whose outer peripheral surface (outer peripheral surface 100) is exposed, a first member (first member 11) that is connected to the gripping member and is movable relative to the gripping member in the axial direction, and a restricting member (restricting member 20) that restricts a relative movable maximum distance between the first member and the gripping member to any of a plurality of values, and
   the outer peripheral surface of the gripping member has a shape including a pair of first planes (first planes 101) that are arranged in a first direction (first direction D1) and are disposed to face each other, and a pair of second planes (second planes 102) that are arranged in a second direction (second direction D2) intersecting the first direction and are disposed to face each other.
   According to (1), since the outer peripheral surface of the gripping member has a shape including four planes, a force can be easily transmitted to the gripping member in a case of gripping and moving the gripping member to move the puncture needle. As a result, it is possible to easily and appropriately conduct the puncture procedure.
(2) The puncture device according to (1),
   in which the first direction and the second direction are orthogonal to each other.
   According to (2), a force can be more easily transmitted to the gripping member.
(3) The puncture device according to (2),
   in which the gripping member has a tubular shape,
   the first member is inserted into a distal end side of the gripping member, and
   an outer peripheral surface (outer peripheral surface 110) of the first member has a shape including a plane (the first plane 111 and the second plane 112) parallel to at least one of the pair of first planes or the pair of second planes.
   According to (3), the outer peripheral surface of the first member and the outer peripheral surface of the gripping member are parallel to each other. Therefore, for example, in a case of placing one hand on the parallel portion and gripping both the first member and the gripping member, the grip can be easily performed. As a result, it is possible to improve the operability in a case where the first member and the gripping member are moved relative to each other.
(4) The puncture device according to (3),
   in which the outer peripheral surface of the first member has a shape including planes (the first plane 111 and the second plane 112) parallel to each of the pair of first planes and each of the pair of second planes.
   According to (4), the first member and the gripping member each have an outer peripheral surface shape having four planes. Therefore, for example, in a case of gripping both the first member and the gripping member with one hand, the grip can be easily performed. As a result, it is possible to improve the operability in a case where the first member and the gripping member are moved relative to each other.
(5) The puncture device according to (4),
   in which the operation part includes a second member (second member 12) that is connected to the first member and is movable relative to the first member in the axial direction, and a fixing member (fixing member 30) that fixes a relative position between the first member and the second member to any of a plurality of positions, and
   an outer peripheral surface (outer peripheral surface 120) of the second member has a shape including a plane (the first plane 121 and the second plane 122) parallel to each of the pair of first planes and the pair of second planes.
   According to (5), since the outer peripheral surface of the second member has a shape including four planes, the mounting can be stably performed in a case where the distal end part of the second member is mounted on the treatment tool introduction hole of the ultrasonic endoscope.
(6) The puncture device according to (5),
   in which the first member has a tubular shape, and
   the second member is inserted into a distal end side of the first member.
   According to (6), the outer diameter increases in the order of the second member, the first member, and the gripping member, which makes it easy to intuitively recognize a positional relationship between the first member and the second member, and a positional relationship between the first member and the gripping member, and the operability can be improved. Further, the gripping member can be made sufficiently thick regardless of the size of the treatment tool introduction hole of the ultrasonic endoscope, which makes it easy to move the puncture needle, and the operability can be improved. Moreover, since the outer peripheral surface of the gripping member having the largest outer diameter includes four planes, it is possible to prevent the operation part from rolling in a case where the operation part is placed on a placement surface such as a desk.
(7) The puncture device according to (6),
   in which a distal end part (distal end part 12t) of the second member is mountable in a treatment tool introduction hole (treatment tool introduction hole 93h) of the ultrasonic endoscope, and
   an outer diameter (outer diameter d2) of the distal end part is larger than an inner diameter (inner diameter d1) of the first member.
   According to (7), since the distal end part of the second member is thicker than a portion that is inserted into the first member, the distal end part can be mounted on the ultrasonic endoscope by being stably gripped even in an overall elongated operation part. As a result, the puncture procedure can be efficiently performed.
(8) The puncture device according to any one of (1) to (7),
   in which the restricting member includes a movable member (movable member 22) that is attached to an outer peripheral surface of the first member to be movable along the axial direction with respect to the first member, and a first rotational movement member (first rotational movement member 21) that is rotationally movable with respect to the movable member, and
   an outer peripheral surface (outer peripheral surface 210) of the first rotational movement member has a shape including a pair of third planes (third planes 213) that are arranged in a third direction (third direction D3) and are disposed to face each other, and a pair of fourth planes (fourth planes 214) that are arranged in a fourth direction (fourth direction D4) intersecting the third direction and are disposed to face each other.
   According to (8), since a force can be easily transmitted to the first rotational movement member in a case where the first rotational movement member of the restricting member is gripped and rotated with the fingers, the puncture procedure can be efficiently performed.
(9) The puncture device according to any one of (5) to (7),
   in which the fixing member includes a second rotational movement member (second rotational movement member 32) that is rotationally movable about a rotational movement shaft (screw portion 31) extending in a direction intersecting the axial direction, and
   a shape of an outer peripheral surface (outer peripheral surface 320) of the second rotational movement member is a shape including a pair of fifth planes (fifth planes 325) that are arranged in a fifth direction (fifth direction D5) intersecting the rotational movement shaft and are disposed to face each other, and a pair of sixth planes (sixth planes 326) that are arranged in a sixth direction (sixth direction D6) intersecting the fifth direction and are disposed to face each other.
   According to (9), since a force can be easily transmitted to the second rotational movement member in a case where the second rotational movement member of the fixing member is gripped and rotated with the fingers, the puncture procedure can be efficiently performed.
(10) The puncture device according to any one of (1) to (9),
   in which a length of each of the pair of first planes and a length of each of the pair of second planes coincide with each other in a state of being viewed in the axial direction.
   According to (10), a force can be easily transmitted to the gripping member regardless of the grip posture of the gripping member.
(11) A puncture device comprising:
   a puncture needle (puncture needle 7); and
   an operation part (operation part 5) that moves the puncture needle in an axial direction and is attachable to and detachable from an ultrasonic endoscope,
   in which the operation part includes a gripping member (gripping member 10) that is interlocked with the puncture needle, a first member (first member 11) that is connected to the gripping member and is movable relative to the gripping member in the axial direction, and a restricting member (restricting member 20) that restricts a relative movable maximum distance between the first member and the gripping member to any of a plurality of values,
   the restricting member includes a movable member (movable member 22) that is attached to an outer peripheral surface of the first member to be movable along the axial direction with respect to the first member, and a first rotational movement member (first rotational movement member 21) that is rotationally movable with respect to the movable member, and
   an outer peripheral surface (outer peripheral surface 210) of the first rotational movement member has a shape including a pair of planes (third planes 213) that are disposed to face each other, and a pair of planes (fourth planes 214) that are arranged in a direction intersecting a direction in which the pair of facing planes are arranged and that are disposed to face each other.

### Explanation of References

3: puncture device
5: operation part
7: puncture needle
7a: inside
7h: opening portion
10: gripping member
10b: sunken portion
11: first member
11b: distal end attachment portion
12: second member
12t: distal end part
12m: luer lock
13: stylet
14: proximal end fixing member
15: insertion body
15t: distal end part
19: flexible sheath
19a: insertion path
20: restricting member
21: first rotational movement member
21b: screw portion
22: movable member
22h: graduation opening portion
30: fixing member
31: screw portion
32: second rotational movement member
50: right hand
51: thumb
52: index finger
53: middle finger
54: ring finger
55: little finger
56: palm
60: left hand
90: ultrasonic endoscope
93: endoscope operation part
93h: treatment tool introduction hole
94: endoscope insertion part
95: distal end part
97: optical imaging mechanism
98: ultrasonic scanning mechanism
100, 120, 210, 320: outer peripheral surface
101, 111, 121: first plane
102, 112, 122: second plane
213: third plane
214: fourth plane
325: fifth plane
326: sixth plane
D 1: first direction
D2: second direction
D3: third direction
D4: fourth direction
D5: fifth direction
D6: sixth direction
CL: axis
P: target tissue

## Claims

1. A puncture device comprising:
a puncture needle; and
an operation part that moves the puncture needle in an axial direction and is attachable to and detachable from an ultrasonic endoscope,
wherein the operation part includes a gripping member that is interlocked with the puncture needle, a first member that is connected to the gripping member and is movable relative to the gripping member in the axial direction, and a restricting member that restricts a relative movable distance between the first member and the gripping member, and
an outer peripheral surface of the gripping member includes a pair of first planes that are arranged in a first direction and are disposed to face each other, and a pair of second planes that are arranged in a second direction intersecting the first direction and are disposed to face each other.

2. The puncture device according to claim 1,
wherein the first direction and the second direction are orthogonal to each other.

3. The puncture device according to claim 2,
wherein the gripping member has a tubular shape,
the first member is inserted into a distal end side of the gripping member, and
an outer peripheral surface of the first member has a shape including a plane parallel to at least one of the pair of first planes or the pair of second planes.

4. The puncture device according to claim 3,
wherein the outer peripheral surface of the first member has a shape including planes parallel to each of the pair of first planes and to each of the pair of second planes.

5. The puncture device according to claim 4,
wherein the operation part includes a second member that is connected to the first member and is movable relative to the first member in the axial direction, and a fixing member that fixes a relative position between the first member and the second member to any of a plurality of positions, and
an outer peripheral surface of the second member has a shape including a plane parallel to each of the pair of first planes and the pair of second planes.

6. The puncture device according to claim 5,
wherein the first member has a tubular shape, and
the second member is inserted into a distal end side of the first member.

7. The puncture device according to claim 6,
wherein a distal end part of the second member is mountable in a treatment tool introduction hole of the ultrasonic endoscope, and
an outer diameter of the distal end part is larger than an inner diameter of the first member.

8. The puncture device according to claim 1,
wherein the restricting member includes a movable member that is attached to an outer peripheral surface of the first member to be movable along the axial direction with respect to the first member, and a first rotational movement member that is rotationally movable with respect to the movable member, and
an outer peripheral surface of the first rotational movement member has a shape including a pair of third planes that are arranged in a third direction and are disposed to face each other, and a pair of fourth planes that are arranged in a fourth direction intersecting the third direction and are disposed to face each other.

9. The puncture device according to claim 5,
wherein the fixing member includes a second rotational movement member that is rotationally movable about a rotational movement shaft extending in a direction intersecting the axial direction, and
a shape of an outer peripheral surface of the second rotational movement member is a shape including a pair of fifth planes that are arranged in a fifth direction intersecting the rotational movement shaft and are disposed to face each other, and a pair of sixth planes that are arranged in a sixth direction intersecting the fifth direction and are disposed to face each other.

10. The puncture device according to any one of claims 1 to 9,
wherein a length of each of the pair of first planes and a length of each of the pair of second planes coincide with each other in a state of being viewed in the axial direction.

11. A puncture device comprising:
a puncture needle; and
an operation part that moves the puncture needle in an axial direction and is attachable to and detachable from an ultrasonic endoscope,
wherein the operation part includes a gripping member that is interlocked with the puncture needle, a first member that is connected to the gripping member and is movable relative to the gripping member in the axial direction, and a restricting member that restricts a relative movable distance between the first member and the gripping member,
the restricting member includes a movable member that is attached to an outer peripheral surface of the first member to be movable along the axial direction with respect to the first member, and a first rotational movement member that is rotationally movable with respect to the movable member, and
an outer peripheral surface of the first rotational movement member includes a pair of planes that are disposed to face each other, and a pair of planes that are arranged in a direction intersecting a direction in which the pair of facing planes are arranged and that are disposed to face each other.
